Europäisches Patentamt

European Patent Office

Office européen des brevets

(19)

(11) Publication number: **0 234 051 B1**

## (12) EUROPEAN PATENT SPECIFICATION

(45) Date of publication of patent specification: **11.12.91**

(51) Int. Cl.5: **C12N 9/72**, C12N 15/58, A61K 37/47

(21) Application number: **86200223.5**

(22) Date of filing: **17.02.86**

(54) **Tissue-type plasminogen activator mutants; recombinant genetic information coding therefor and process for preparing said mutants; their use and pharmaceutical compositions.**

(43) Date of publication of application:
**02.09.87 Bulletin 87/36**

(45) Publication of the grant of the patent:
**11.12.91 Bulletin 91/50**

(84) Designated Contracting States:
**AT BE CH DE FR GB IT LI LU NL SE**

(56) References cited:
| | |
|---|---|
| EP-A- 207 589 | EP-A- 213 794 |
| EP-A- 227 462 | EP-A- 0 093 619 |
| WO-A-87/03906 | WO-A-87/04722 |

GENE, Vol. 33, n 3, 1985, pages 279-284.
Elsevier Science Publishers, Amsterdam
(NL). M.J. Browne et al "Isolation of a human
tissue-type plasminogen-activator genomic
DNA clone and its expression in mouse L
cells"

(73) Proprietor: **Stichting Centraal Laboratorium
van de Bloedtransfusiedienst van het Neder-
landse Rode Kruis
Plesmanlaan 125
NL-1066 CX Amsterdam(NL)**

(72) Inventor: **Pannekoek, Hans
Stommeerweg 36
NL-1431 EW Aalsmeer(NL)**
Inventor: **Veerman, Arnoldus Johannes Gerar-
dus
Burgemeester van Baarsstraat 40A
NL-1131 WV Volendam(NL)**
Inventor: **Van Zonneveld, Anton Jan
Medusastraat 25A
NL-2315 GR Leiden(NL)**

(74) Representative: **Smulders, Theodorus A.H.J.,
Ir. et al
Vereenigde Octrooibureaux Nieuwe Parklaan
107
NL-2587 BP 's-Gravenhage(NL)**

## Description

The present invention relates to tissue-type plasminogen activator (t-PA) mutants having improved binding characteristics towards fibrin compared to naturally occurring t-PA. This invention also relates to a process for preparing said mutants by genetic engineering technology and to the recombinant genetic information used therein. Finally, the invention also relates to the use of said mutants in therapy or imaging and to pharmaceutical compositions which are suitable for said purposes.

Tissue-type plasminogen activator (t-PA) is a serine protease which converts the zymogen plasminogen into plasmin, a serine protease which degrades the fibrin network (1). The activity of t-PA is substantially accelerated in the presence of fibrin, a property which has focussed the attention on t-PA as a potential therapeutic anti-thrombogenic agent (2-4). The molecular mechanism of the stimulatory influence of fibrin is based on the notion that both the substrate plasminogen and the enzyme t-PA are bound to and aligned on the fibrin matrix, facilitating a localized generation of plasmin.

t-PA is synthesized and secreted by vascular endothelial cells as a single-chain polypeptide (5). This molecule is converted by plasmin or trypsin into a two-chain polypeptide connected by a disulfide bond. The heavy (H) chain of t-PA ($M_r$ 39 000) is located at the amino-terminus, whereas the light (L) chain ($M_r$ 33 000) is at the carboxy-terminal end (6). Based on the amino-acid sequence homology of t-PA with both the trypsin family of serine proteases, prothrombin, epidermal growth factor and fibronectin, a model for the secondary structure of t-PA has been proposed (6,7). In this model, different structural domains have been assembled to create a composite mosaic polypeptide. The L-chain of t-PA was proposed to harbor the serine protease moiety, responsible for plasminogen activator activity. Recently, we found evidence which demonstrates that in mammalian cells separately expressed t-PA L-chain cDNA is indeed solely capable to convert plasminogen into plasmin and this activity is not accelerated by fibrin. The H-chain of t-PA contains considerable amino-acid homology with several plasma proteins. Apart from a typical signal peptide (S) and a prosequence (P), similar to those of serum albumin (8,9), one can distinguish a 'finger' domain (F) resembling the regions on fibronectin involved in fibrin binding (10,11) and a structure partially homologous to both human and mouse epidermal growth factor (E) (12,13). Furthermore, two 'kringle' structures are proposed to be situated on the carboxy-terminal part of the H-chain, highly homologous to structural and functional domains on plasminogen, shown to be involved in fibrin binding of plasminogen (14).

The elucidation of the chromosomal structure of the t-PA gene and the alignment with the complete amino-acid sequence revealed in intriguing observation, namely that exons or sets of exons coincide with the proposed structural domains (6,15,16). Those studies and others have led to the hypothesis that mosaic proteins, such as t-PA and other plasma proteins, are composed of different functional 'modules' as a result of 'exon shuffling', an evolutionary rearrangement event (17,18).

Herein, we provide evidence that structural domains in the t-PA protein, encoded by separate exons or sets of exons, harbor autonomous functions.

Based thereon, this invention provides tissue-type plasminogen activator (t-PA) deletion mutants having improved binding strength, binding selectivity, or both, towards fibrin, said mutants comprising at least the native light chain (L) and either the native finger domain (F), or the native kringle 2 domain (K2), or both, and lacking at least the complete native kringle 1 domain (K1).

Some particular embodiments of tissue-type plasminogen activator deletion mutants of the invention are:

mutants comprising the native L chain and the native K2 domain, and lacking at least part of the native F domain, at least part of the native epidermal growth factor domain (E) and the complete native K1 domain;

mutants comprising the native L chain, the native F domain and the native K2 domain, and lacking at least part of the native E domain and the complete native K1 domain; and

mutants comprising the native L chain and the native F domain, and lacking at least part of the native E domain, at least part of the native K2 domain and the complete native K1 domain.

Further, this invention provides recombinant genetic information in the form of RNA, single strand DNA or double strand DNA encoding either a t-PA deletion mutant as defined above, or a precursor of such t-PA deletion mutant which comprises the native signal peptide (S), the native propeptide (P), or both, at the N-terminus. The invention also covers a recombinant cloning and/or expression vector, comprising a suitable cloning and/or expression vehicle having inserted therein recombinant genetic information as defined above.

This invention provides a process for preparing a t-PA deletion mutant as defined herein by growing a suitable host microorganism or cell culture, which has been transformed by a recombinant expression vector comprising a suitable expression vehicle having inserted therein recombinant genetic information encoding said t-PA deletion mutant or a precursor thereof which comprises the native signal peptide (S), the native propeptide (P), or both, at the N-terminus, and isolating the t-PA deletion mutant produced therein.

The invention covers a microorganism or cell culture which has been transformed by a recombinant vector as defined herein and is capable of cloning and/or expressing the recombinant genetic information as defined herein.

This invention also covers a pharmaceutical composition for use in therapy for venous thrombosis, arterial thrombosis, myocardial infarction, lung emboly, and other conditions of fibrin clot arrested blood circulation, comprising a t-PA deletion mutant as defined herein and at least one pharmacologically acceptable carrier, diluent or vehicle.

Preferably, said pharmaceutical composition comprises an aqueous solution of a t-PA deletion mutant as defined above, which is suitable for intravenous, intraarterial, or intracoronarial administration.

Several published documents describe t-PA deletion mutants.

WO 87/03906 published 2 July 1987 and partly based on an earlier priority application, discloses t-PA mutants prepared from recombinant DNA constructs containing unique restriction sites inserted into the coding sequence between the respective domains. As a result thereof, the t-PA mutants have modified domains rather than native domains. For example, the F domain contains an additional Arg residue inserted between Ser(1) and Tyr(2), and the K2 domain comprises the sequence SerValAsn instead of the native Asn(177)Ser(178).

EP 0 207 589 published 7 January 1987 and partly based on an earlier priority application, discloses a t-PA deletion mutant lacking the complete E domain, i.e. from Cys(51) to Asp(87), inclusive. The K1 domain is not lacking.

EP 0 213 794 published 11 March 1987 and partly based on an earlier priority application, discloses hybrid plasminogen activators which contain plural, heterologous polypeptide kringles. These mutants comprise a part derived from t-PA, and another part derived from, e.g., urokinase or prothrombin.

WO 87/04722 published 13 August 1987 and partly based on an earlier priority application, discloses several kinds of t-PA mutants, viz.:

(A) deletion mutants having an N-terminal deletion comprising the complete E domain Cys(51)-Asp(87), or almost the complete F domain Cys-(6)-Ser(50), or both Cys(6)-Ile(86);
(B) mutants comprising one or several modified glycosylation sites;
(C) mutants having a modified cleaving site Arg-(275)-Ile(276).

EP 0 227 462 published 1 July 1987 and partly based on an earlier priority application, discloses partly similar t-PA mutants, viz .:

(A) deletion mutants having an N-terminal deletion of 1 to 5 amino acids or a C-terminal deletion of 1-25 amino acids (i.e. mutants lacking a few amino acids at the N-terminus of the F domain or lacking a part of the C-terminus of the L chain);
(B) mutants comprising one or several modified glycosylation sites (as in WO 87/04722);
(C) mutants having a modified cleaving site (as in WO 87/04722).

Mutants of groups B and C are not deletion mutants as they contain modified sites. The mutants disclosed in these documents always contain the complete K1 domain.

EP 0 196 920 published 8 October 1986 and partly based on an earlier priority application, discloses a t-PA mutant isolated from recombinant Bowes melanoma cells which t-PA mutant consists of the amino acids Ala(160)-Pro(527). Recombinant DNA manipulations, recombinant plasmids etc. are not described. This t-PA mutant lacks a part of the K1 domain only: the amino acids 160-174 belonging to the K1 domain are not absent therefrom.

## Materials and methods

### Materials

Restriction endonucleases, T4 DNA ligase, T4 DNA polymerase, T4 polynucleotide kinase and Escherichia coli DNA polymerase I (Klenow fragment) were purchased from New England Biolabs. Mung Bean nuclease was from P.L. Biochemicals Inc., Tunicamycin from Calbiochem, Iscove's modified medium from Flow Laboratories and all reagents required for the plasminogen activator assay were from Kabi Vitrum.

### General methods

Plasmid DNA was isolated by a modification of the alkaline--lysis procedure (19), followed by CsCl-equilibrium centrifugation. Enzyme reactions were carried out using standard conditions (20). Nucleotic sequence determinations were performed using the method of Maxam and Gilbert (21).

### Construction of expression plasmids

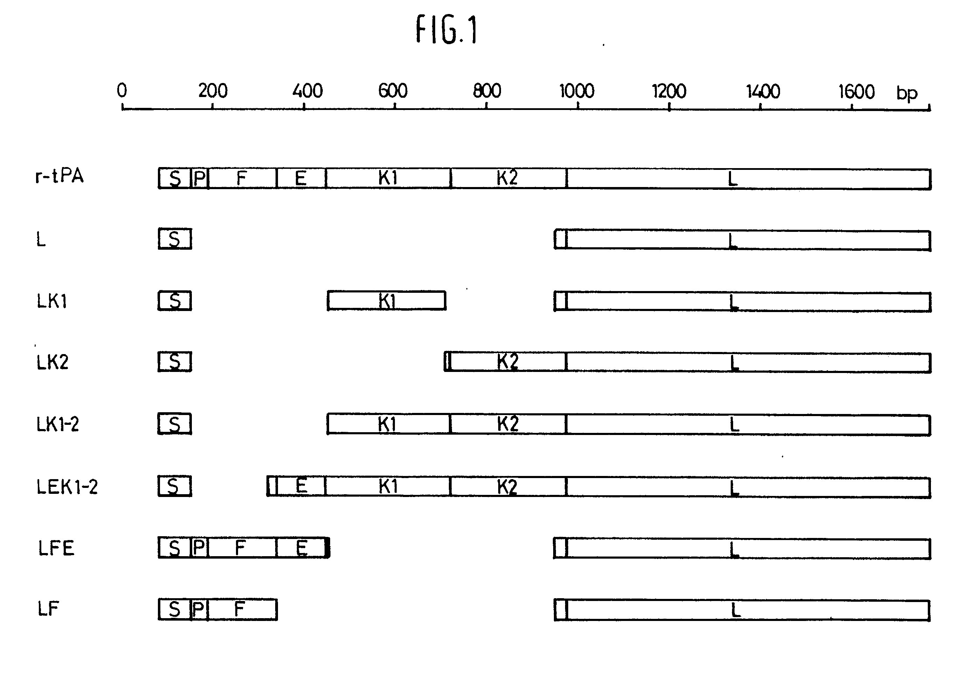

Plasmid ptPA8FL consists of vector pBR322 and full-length t-PA cDNA (2,540 bp) inserted by G-C tailing into the PstI site of the vector. Human t-PA cDNA was constructed using Bowes Melanoma polyA$^+$RNA. A fragment (2,087 bp) of the full length t-PA cDNA, extending from bp 78 to 2,165 (numbering of the t-PA cDNA is according to Pennica et al. (6)) and containing the entire coding sequence, was subcloned between the HincII and BamHI sites of the pUC9 polylinker, resulting in plasmid pUCtPA. A HindIII-SacI fragment of pUCtPA was inserted together with a SacI-BglII fragment of ptPA8FL into the HindIII and BglII sites of PSV2 (22) to yield pSV2tPA. This plasmid contains the origin of replication and the $\beta$-lactamase gene of pBR322, the SV40 early promotor, the t-PA-coding sequence and SV40 splice and polyadenylation sequences. All t-PA deletion-mutant expression plasmids were derived from pSV2tPA by constructing deletions in the coding sequence using restriction sites adjacent to the junctions of the hypothetical domains. To preserve the translation reading frame, in some of the constructions restriction fragment termini were altered using DNA-modifying enzymes (details are given in the legend to Fig.1). DNA-sequence determinations were performed to verify a correct in-phase ligation of all new cDNA junctions in each construct. We made a deposit on February 6, 1986 with the Centraalbureau voor Schimmelcultures (CBS), Baarn, The Netherlands, of an E. coli K12DH1 containing the plasmid pSV2/t-PA.

Tissue culture and transfection

Mouse Ltk⁻cells were maintained in Iscove's modified minimal medium, containing penicillin, streptomycin and 10% fetal calf serum. Transfection was carried out essentially as described (23). After transfection, the cells were incubated in serum-free Iscove's medium, containing penicillin, streptomycin and 0.3 mg/ml acid-treated bovine serum albumin. In some of the experiments, tunicamycin (1 $\mu$g/ml) was added. Five days after transfection, cell media regularly contained 1 to 5 $\mu$g rt-PA or rt-PA mutant proteins per $8 \times 10^6$ cells.

Gelatin-plasminogen gelelectrophoresis

Electrophoresis on gelatin gels, containing 13 $\mu$g/ml copolymerized plasminogen, was performed essentially as described (24). Ten per cent polyacrylamide gels, containing 0.1% sodium dodecylsulfate, were incubated after electrophoresis in 2.5% Triton-X 100 for 2 h to remove sodium dodecylsulfate. Subsequently, the gels were incubated for 6 h in 0.1 M glycin-NaOH (pH 8.3). Gelatin degradation, due to plasminogen activation in the gel, was visualized by contrastaining using 0.1% amidoblack.

Plasminogen activator assay

Plasminogen activator activity was determined using an indirect spectrophotometric assay (27). This assay measures the amidolytic activity of plasmin, which is generated by the plasminogen activator-catalyzed conversion of plasminogen and is based on the hydrolysis of a bond in the chromogenic substrate D-val-leu-lys pNitro anilide (S2251). Assays were performed at 37° C in 250 $\mu$l 0.1 M Tris HCl (pH 7.5), containing 0.13 $\mu$M plasminogen, 0.1% Tween-80, 120 $\mu$g/ml CNBr-digested fibrinogen (when indicated) and 0.30 mM S2251. The absorbance at 405 nM was followed for 6h.

Immunoradiometric assay for t-PA L-chain antigen

Goat-anti-mouse IgG coupled to Sepharose beads was incubated with limiting amounts of the murine monoclonal anti-human t-PA L-chain IgG (ESP2) (25) and with a $^{125}$I-radiolabeled t-PA tracer in phosphate-buffered saline, containing 1% bovine serum albumin, 0.1% Tween-80 and 10 mM EDTA. The incubation was performed end over end for 18 h at room temperature in stoppered polystyrene tubes. Separation of bound and free radiolabeled tracer was carried out by centrifugation for 2 min at 3000xg. The Sepharose beads were washed 4 times with 2 ml 0.9 M NaCl, 0.1% Tween-80, 10 mM EDTA and bound radioactivity was determined in a gamma-counter. Unlabeled Bowes Melanoma t-PA was used as a competitor for the binding of $^{125}$I-t-PA. Approximately 100 ng t-PA, in a final volume of 0.5 ml, reduced the binding of the tracer till 50% of the maximum value. Conditioned media, containing rt-PA or rt-PA mutant protein, were also used as competitor for radiolabeled t-PA to bind to the monoclonal ESP2 IgG. A comparison between the percentage of competition of the conditioned media and serial dilutions of Bowes Melanoma t-PA allows an estimation of the amount of L-chain antigen present.

Results

Construction of t-PA deletion-mutant expression plasmids

To study the biological properties of the separate hypothetical domains of t-PA, we have constructed a series of t-PA cDNA expression plasmids which systematically lack the coding sequence of one ore more of the domains on the H-chain. For that purpose, we have employed our cloned full-length t-PA cDNA, composed of a 95-bp 5′-untranslated region, a 1,686-bp-coding region and a 759-bp 3′-untranslated region. Restriction-enzyme analysis and partial nucleotide-sequence determinations revealed identity with the DNA sequence reported by Pennica et al.(6). Sections of full-length t-PA cDNA were inserted into a 'shuttle vector' (pSV2), consisting of parts of plasmid pBR322 and the eukaryotic virus SV40. In these constructs, t-PA cDNA is preceded by the SV40 early promoter and linked at its 3′ end to splice and polyadenylation signals. All constructs have two features in common. First, they harbor the coding sequence for the t-PA signal peptide (nucleotides 85 till at least 156) to ensure secretion of the (mutant) polypeptides. Second, all constructs contain the entire coding region for the t-PA L-chain (nucleotides 953 till 1,771). The latter domain is responsible for the plasminogen activator activity, thereby allowing a convenient assay of the biological activity. Furthermore, the involvement of the different domains of the H-chain on the stimulation of the plasminogen activator activity of t-PA by fibrin can be readily assessed. Essential junctions of the constructs were sequenced to verify the continuity of the translation reading frame. A schematic representation of the H-chain t-PA cDNA-deletion mutants is shown in Fig. 1.

Characterization of the expression products

Mouse Ltk⁻ cells appeared to be suitable hosts for transient expression of rt-PA, programmed by t-PA cDNA-harboring plasmids, because these cells can be efficiently transfected and do neither contain nor secrete plasminogen activator(s). The different expression products, present in the serum-free media 5 days after transfection, were analyzed by gelatin-plasminogen gel electrophoresis (not shown). This technique localizes plasminogen activator activity in SDS-polyacrylamide gels. The method depends on the principle that plasminogen and gelatin, when incorporated into the polyacrylamide matrix at the time of casting, are retained during electrophoresis. In-situ plasmin formation and consequently degradation of gelatin can be visualized by negative staining. Clearly, Melanoma t-PA and rt-PA display similar mobilities in this gel system. The rt-PA-deletion mutants were also secreted, apparently due to routing directed by the signal peptide. The mobility of the deletion-mutant proteins corresponds with the expected values. Obviously, all mutant proteins exhibit a basal plasminogen activator activity in accord with our previous observations that the L-chain suffices for this activity. To demonstrate that the heterogeneity of the products results from differential glycosylation, tunicamycin, an inhibitor of N-glycosylation, was added to the cell media during the expression of the products. The results (not shown) were that under these conditions all the proteins display single bands on the gelatin gel, indicating that they are expressed as unique polypeptides. Our data and those of others (26) show that the carbohydrate moieties of t-PA are not involved in its biological activity. We consider the expression products to be valuable tools in studying the biological properties of the as yet hypothetical domains of the t-PA molecule.

Plasminogen activation by the mutant proteins; the influence of fibrinogen fragments

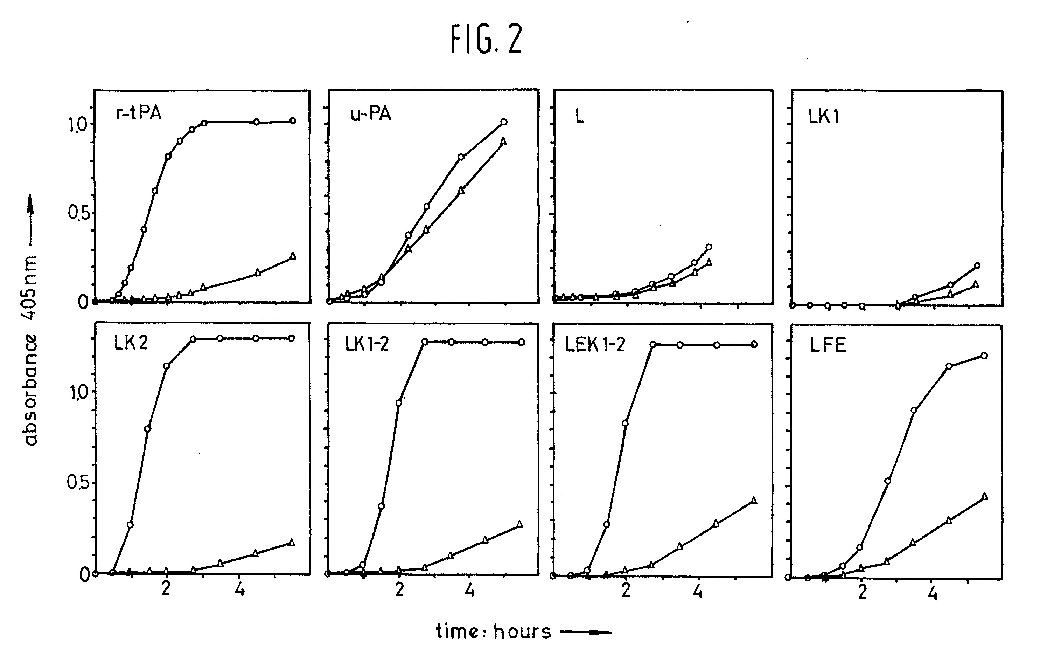

To investigate the effect of fibrin on the plasminogen activator activity of the deletion-mutant proteins, we used an amidolytic assay, relying on the chromogenic substrate S2251. We determined the activity of rt-PA and rt-PA mutant proteins in the presence and absence of cyanogen-bromide-digested fibrinogen, a digest known to mimic the potentiating the effect of fibrin (27). The data are given in Fig. 2. In the absence of fibrinogen fragments, rt-PA only has a basal activity, but upon adding fibrinogen fragments, the plasminogen activator activity is greatly accelerated. The mutant proteins L and LK1 display only a basal activity, and no significant stimulation of the plasminogen activator activity by fibrinogen fragments was detected. In contrast, the plasminogen activator activity of the mutant proteins LEK 1-2, LK1-2 and LK2 was stimulated by fibrinogen fragments to the same magnitude as rt-PA. The plasminogen activator activity of the mutant proteins LFE and LF (not shown) were also stimulated by fibrinogen fragments, but to a lesser extent than the K2-domain-containing mutant proteins. These results indicate that the K2 domain of the t-PA H-chain and, to a lesser extent, the finger (F) region mediate the stimulatory effect of fibrin. Finally, 'kringle' K1 and the EGF region apparently do not contribute to the stimulatory mechanism. It is assumed that the molecular basis for the stimulation by fibrin is that both plasminogen and t-PA are bound by the fibrin polymer, hereby aligning t-PA with its substrate (3,28). Therefore, we examined the fibrin-binding properties of the t-PA deletion-mutant proteins.

Fibrin binding

Fibrin matrices were formed in the presence of either rt-PA or rt-PA deletion-mutant proteins and subsequently pelleted (29). Plasminogen activator activity of supernatants and of solubilized pellets were analyzed on gelatin-plasminogen gels (not shown). In the case of rt-PA and L, the total amount used (input), the non-binding and the binding fractions were investigated. Most of the rt-PA-input fraction was bound to the fibrin matrix, whereas no significant binding of the L-chain protein was detected. The input and the fraction bound to fibrin of the other proteins were also investigated. LK1 does not exhibit significant binding to fibrin. The K2-domain-containing mutants LEK1-2, LK1-2 and LK2 bound to fibrin to about the same extent, although not as efficient as the binding of rt-PA. The LFE and LF mutants also bound to fibrin, but to a lesser extent than the K2-domain-containing mutant proteins. Hence, the stimulatory effect of fibrinogen fragments on the plasminogen activator activity, mediated by kringle K2 and to a lesser extent by the F region, correlates well with the fibrin-binding characteristics of these domains. Apparently, the acceleration of the plasminogen activator activity of t-PA is intimately linked to binding to the fibrin matrix.

Discussion

The elucidation of the t-PA gene structure by Ny et al. (15) revealed that separated exons or separate sets of exons encode structural domains, which were postulated on the basis of homology with other plasma proteins. To test whether these autonomous structural domains indeed have autonomous functions, we have expressed a series of rt-PA deletion-mutant proteins lacking one or more of the structural domains and studied the remaining biological properties. Earlier studies from this laboratory showed that separately expressed L-chain molecules harbor the plasminogen activator activity, however, this activity could not be stimulated by fibrin. In those studies, the L-chain molecules were not secreted by the tissue-culture cells that were used for expression of L-chain cDNA. In this paper, we have been able to show that efficient secretion of the expression products occurs, provided the signal peptide is encoded at the 5′ end of the cDNA of different deletion mutants. This result indicates that the t-PA signal peptide has an autonomous function.

The finger (F) domain exhibits a fibrin-binding function and also mediates in part the stimulatory effect of 'fibrin' on plasminogen activator activity of t-PA.

Clearly, the K2 domain has an autonomous function in the binding of fibrin as well as in mediating stimulation of the plasminogen activator activity of t-PA by fibrin. Surprisingly, the kringle K1 domain does not seem to be involved in the fibrin-binding property of t-PA. Although, the amino-acid sequence of kringle K1 and K2 are highly homologous and the secondary structure might be similar (6,7), apparently subtle differences may specify fibrin affinity. Such differences might explain the lack of fibrin affinity of urokinase (u-PA), in spite of the presence of a kringle structure on this molecule.

We propose that the fibrin-binding characteristics of t-PA are mediated both by the finger domain (F) and the K2 domain, the latter contributing most to the binding. Additional evidence for the involvement of the kringle K2 domain to fibrin binding was gained by the observation that a murine monoclonal anti-human t-PA antibody (ESP5) (25), which was found to inhibit in part fibrin binding of t-PA, specifically bound to LK2, but not to the L-chain product (results not shown). The fact that this conformation-dependent monoclonal antibody reacted equally well with rt-PA as with the K2-domain-containing mutant proteins indicates that the K2 domain retains its native conformation in the deletion-mutant proteins.

Mosaic proteins (e.g.t-PA) have been proposed to be evolved by 'exon shuffling', an evolutionary process linking different functional structures. By showing that structural domains in t-PA, encoded by separate exons or sets of exons, are autonomous functional domains, our study supports the concept of exon shuffling as a mechanism to create new genes. In our view, the approach explored here to investigate the structure-function relationships of the t-PA molecule with these and other deletion mutants may elucidate which domains are involved in the complex formation of t-PA with plasminogen activator inhibitor(s) (30,31) and in the binding of t-PA to the receptor(s) in the liver (32,33), responsible for the rapid clearance of t-PA from the bloodstream.

Summarizing, we employed transfected mouse LtK⁻ cells for transient expression of recombinant human tissue-type plasminogen activator (rt-PA) or of rt-PA deletion-mutant proteins, encoded by SV40-pBR322-derived t-PA cDNA plasmids. The t-PA cDNA-deletion mutants have two features in common, i.e. cDNA programming the signal peptide and the coding region for the L-chain. Consequently, rt-PA mutant proteins are efficiently secreted and display plasminogen activator activity. The N-terminal H-chain, being an array of structural domains homologous to other plasma proteins ('finger', 'epidermal growth factor', 'kringles'), was mutated using restriction endonucleases to delete one or more structural domains. The stimulatory effect of fibrinogen fragments on the plasminogen activator activity of t-PA was demonstrated to be mediated by the kringle K2 domain and to a lesser extent by the finger region, but not by the kringle K1 and the EGF domains. These data correlate well with the fibrin-binding properties of the rt-PA deletion-mutant proteins, indicating that the stimulation of the activity by fibrinogen fragments is based on aligning the substrate plasminogen and t-PA on the fibrin matrix. Our results also support the evolutionary concept of 'exon shuffling', arranging structural domains which constitute autonomous functions of the protein.

Our work as described above provides the understanding and tools necessary for the designing and actual production of t-PA mutants having improved binding characteristics towards fibrin compared to naturally occurring t-PA forms and possibly to t-PA mutants having more desirable properties in the complex formation with plasminogen activator inhibitor(s) and/or the binding to the receptor(s) in the liver.

A direct result of our work is the development of pharmaceutical compositions comprising t-PA mutants having improved binding characteristics towards fibrin, which can be beneficial in therapy for conditions of fibrin clot arrested blood circulation, such as, for example, venous thrombosis, arterial thrombosis, myocardial infarction and lung emboly. Of course the t-PA mutants used for therapy purposes must at least comprise the L-chain which harbors the plasminogen activator activity. This requirement does not exist in a second kind of use of novel t-PA mutants having improved binding characteristics towards fibrin, which is their use in imaging for localizing fibrin clots, such as, for example, a thrombus. Then, however, the mutants should comprise a detectable label, such as, for example, a radio-isotope.

Pharmaceutical compositions containing t-PA are known (4) and men in the art will therefore have no problems whatsoever in formulating pharmaceutical compositions containing t-PA mutants as claimed herein.

Although different administration routes can be employed, normally intravenous, intraarterial or intracoronarial administration will be preferred to obtain quickest results. For these administration routes, the pharmaceutical compositions will normally comprise an aqueous solution of the t-PA mutant, optionally further containing conventional additives, such as, for example, additives which assist in solving the t-PA mutant, as is well-known for aqueous solutions containing t-PA itself.

Legends to Figures

Fig.1 - Schematic representation of t-PA cDNA present in the rt-PA and rt-PA deletion-mutant expression vectors. rt-PA is coded for by the entire coding cDNA. Deletion-mutant cDNAs were constructed by fusing t-PA restriction-fragment termini that were in some cases enzymatically modified to preserve the translation reading frame. L [deletion of base pair 158 till (not including) 953 (Δ 158-953)] was constructed by fusing the EcoRII(153)-terminus [filled in by E.coli DNA polymerase-I large fragment (Klenow)] to the Scal(950)- terminus. LK2 (Δ 156-711); fusion of the BstNI(153)-terminus (Klenow) to the Ddel(710)-terminus (Klenow). LK1-2 (Δ158-458); fusion of the EcoRII(153)-terminus (Klenow) to the HaeIII(456)-terminus. LK1 (Δ 158-458/Δ713-953); as LK1-2 with one extra deletion made by fusion of the Ddel(710)-terminus [partially filled in by Klenow (TTP, dGTP) followed by a Mung Bean nuclease treatment] to the Scal(950)-terminus. LEK1-2 (Δ 158-326); fusion of the EcoRII-(153)-terminus (Klenow) to the DraIII(319)-terminus (T4-DNA polymerase, adding dATP only, followed by a Mung Bean nuclease treatment). LFE ( Δ 455-953); fusion of the BstNI(452)-terminus (Klenow) to the Scal(950)-terminus. LF ( Δ 332-953) has been constructed in two steps. a)LK1-2 was digested with Narl (517) and Scal(950) and treated with Klenow. A Bbvl fragment (252-341) was isolated from pSV2/t-PA DNA digested with Bbvl. This fragment was incubated with T4-DNA polymerase, dATP, dCTP, TTP and subsequently with Mung Beam nuclease. This modified fragment was inserted into LK1-2, prepared as described above. b) The resulting plasmid was digested with DraIII (at 319 and 1365; lacking 332-953). The DraIII fragment, containing the deletion, was employed to substitute for the 'intact' DraIII fragment (319-1365) of pSV2/t-PA to yield LF.

DNA sequencing showed that either T4-DNA polymerase or Mung Bean nuclease has 'nibbled' at the Bbvl ends. S, signal peptide; P, propeptide; F, finger domain; E, EGF domain; K1 and K2, kringle domains, and L, light chain.

Fig.2 - The influence of 'fibrin' (fibrinogen fragments) on the plasminogen activation by rt-PA, u-PA, L, LK1, LK2, LK1-2, LEK1-2 and LFE proteins as determined with an amidolytic assay using the chromogenic substrate S2251 (27). 2 mU u-PA or 20 μl serum-free medium containing about 0.15 pmoles (determined by the immunoradiometric assay) of the different expression products were used. The absorbance at 405 nm was followed for at least 4 h and conditioned serum-free medium of Ltk⁻cells, that were transfected with promotorless ptPA8FL DNA, was used as a control. O, + fibrinogen fragments; Δ, -fibrinogen fragments.

References

1. Collen, D. (1980) Thrombos. Haemostas. 43, 77-89.
2. Wallen, P. (1977) in Thrombosis and Urokinase, eds. Paoletti, R. & Sherry, S. (Academic Press, New York), pp. 91-102.
3. Hoylaerts, M., Rijken, D.C., Lijnen, H.R. & Collen, D.(1982) J. Biol. Chem. 257, 2912-2919.
4.Goeddel, D.V., Kohr, W.J., Pennica, D. & Vehar, G.A. (1983), EP-A-0093619.
5. Rijken, D.C., Wijngaards, G. & Welbergen, J. (1980) Thromb.Res. 18, 815-830.
6. Pennica, D., Holmes, W.E., Kohr, W.J., Harkins, R.N., Vehar, G.A., Ward, C.A., Bennett, W.F., Yelverton, E., Seeburg, P.H., Heyneker, H.L. & Goeddel, D.V. (1983) Nature 301, 214-221.
7. Bányai, L., Váradi, A. & Patty, L. (1983) FEBS Lett. 163, 37-41.

8. Patterson, J.E. & Geller, D.M. (1977) Biochem, Biophys. Res.Comm.74, 1220-1226.

9. Lawn, R.M., Adelman, J., Bock, S.C., Franke, A.E., Houck, C.M., Najarian, R.C., Seeburg, P.H. & Wion, K.L. (1981) Nucleic Acids Res.9, 6103-6114.

10. Sekiguchi, K., Fukuda, M. & Hakamori, S.I. (1981) J.Biol.Chem.256, 6452-6462.

11. Petersen, T.E., Thogersen, H.C., Skorstengaard, K., Vibe-Pedersen., K., Sahl, P., Sottrup-Jensen, L. & Magnusson, S.(1983) Proc.Natl. Acad.Sci. USA 80, 137-141.

12. Savage Jr., C.R., Inagami, T. & Cohen, S. (1972) J. Biol.Chem. 247, 7612-7621.

13. Gregory. H. & Preston, B.M. (1977) Intern.J. Pept. Prot. Res. 9, 107-118.

14. Sottrup-Jensen, L., Claeys, H., Zajdel, M.,Petersen, T.E. & Magnusson, S. (1978) in Progress in Chemical Fibrinolysis and Thrombolysis, eds.Davidson, J.F., Rowan, R.M., Samama, M.M. & Desuoyers, P.C. (Raven Press, New York), vol.3, pp. 191-209.

15. Ny, T., Elgh, F. & Lund, B. (1984) Proc.Natl.Acad.Sci. USA 81, 5355-5359.

16. Doolittle, R.F. (1985) Trends in Biochemical Sciences, June vol., pp. 233-237.

17. Gilbert, W.(1978) Nature 271, 501.

18. Craik, C.S., Sprang, S., Fletterick, R. & Rutter, W.J. (1982) Nature 299, 180-182.

19. Birnboim, H.C. & Doly, J. (1979) Nucleic Acids Res.7 1513-1522.

20. Maniatis, T., Fritsch, E.F. & Sambrook, J. (1982) in Molecular Cloning: A Laboratory Manual (Cold Spring Harbor Laboratory, New York), pp. 1-545.

21. Maxam, A.M. & Gilbert, W. (1980) Methods in Enzymology 65, 499-560.

22. Mulligan, R.C. & Berg, P. (1981) Proc.Natl.Acad.Sci.USA 78, 2072-2076.

23. Lopata, M.A., Cleveland, D.W. & Sollner-Webb,B. (1984) Nucleic Acids Res.12, 5707-5717.

24. Heussen, C. & Dowdle, E.B. (1980) Anal.Biochem 102, 196-202.

25. MacGregor, I.R., Micklem, L.R., James, K. & Pepper, D.S. (1985) Thrombos. Haemostas.53, 45-50.

26. Little, S.P., Bang, N.U., Harms, C.S., Marks C.A. & Mattler, L.E.(1984) Biochemistry 23, 6191-6195.

27. Verheijen, J.H., Mullaart, E., Chang, G.T.G., Kluft, C. & Wijngaards, G. (1982) Thrombos.Haemostas. 48, 266-269.

28. Rijken, D.C. & Collen, D. (1981) J. Biol.Chem. 256, 7035-7041.

29. Loskutoff, D.J. & Mussoni, L. (1983) Blood 62, 62-68.

30. Loskutoff, D.J., Van Mourik, J.A., Erickson, L.A. & Lawrence, D. (1983) Proc.Natl.Acad.Sci.USA 80, 2956-2960.

31. Chmielewska, J., Ranby, M. & Wiman, B. (1983) Thrombos.Haemostas.50, 193.

32. Korninger, C., Stassen, C.M. & Collen, D. (1981) Thrombos. Haemostas. 46, 658-661.

33. Fuchs, H.E., Berger Jr., H. & Pizzo, S.V. (1985) Blood 65, 539-544.

## Claims

1. Tissue-type plasminogen activator (t-PA) deletion mutants having improved binding strength, binding selectivity, or both, towards fibrin, said mutants comprising at least the native light chain (L) and either the native finger domain (F), or the native kringle 2 domain (K2), or both, and lacking at least the complete native kringle 1 domain (K1).

2. Tissue-type plasminogen activator deletion mutants as claimed in claim 1, said mutants comprising the native L chain and the native K2 domain, and lacking at least part of the native F domain, at least part of the native epidermal growth factor domain (E) and the complete native K1 domain.

3. Tissue-type plasminogen activator deletion mutants as claimed in claim 1, said mutants comprising the native L chain, the native F domain and the native K2 domain, and lacking at least part of the native epidermal growth factor domain (E) and the complete native K1 domain.

4. Tissue-type plasminogen activator deletion mutants as claimed in claim 1, said mutants comprising the native L chain and the native F domain, and lacking at least part of the native epidermal growth factor domain (E), at least part of the native K2 domain and the complete native K1 domain.

5. Recombinant genetic information in the form of RNA, single strand DNA or double strand DNA encoding either a t-PA deletion mutant as claimed in any of the claims 1-4, or a precursor of such t-PA deletion mutant which comprises the native signal peptide (S), the native propeptide (P), or both, at the N-terminus.

6. Recombinant cloning and/or expression vector, comprising a suitable cloning and/or expression vehicle having inserted therein recombinant genetic information as claimed in claim 5.

7. A process for preparing a t-PA deletion mutant as claimed in any of the claims 1-4 by growing a suitable host microorganism or cell culture, which has been transformed by a recombinant expression vector comprising a suitable expression vehicle having inserted therein recombinant genetic information encoding said t-PA deletion mutant or a precursor thereof which comprises the native signal peptide (S), the native propeptide (P), or both, at the N-terminus, and isolating the t-PA deletion mutant produced therein.

8. Microorganism or cell culture which has been transformed by a recombinant vector as claimed in claim 6 and is capable of cloning and/or expressing the recombinant genetic information as claimed in claim 5.

9. Pharmaceutical composition for use in therapy for venous thrombosis, arterial thrombosis, myocardial infarction, lung emboly, and other conditions of fibrin clot arrested blood circulation, comprising a t-PA deletion mutant as claimed in any of the claims 1-4 and at least one pharmacologically acceptable carrier, diluent or vehicle.

10. Pharmaceutical composition as claimed in claim 9, comprising an aqueous solution of a t-PA deletion mutant as claimed in any of the claims 1-4, which is suitable for intravenous, intraarterial, or intracoronarial administration.

**Revendications**

1. Mutants de délétion de l'activateur de plasminogène tissulaire (t-PA), ayant une résistance ou une sélectivité de liaison améliorée, ou les deux, vis-à-vis de la fibrine, lesdits mutants comprenant au moins la chaîne légère native (L) et soit le domaine natif "doigt" ("finger") (F), soit le domaine natif "kringle 2" (K2), ou les deux, et étant dépourvus d'au moins le domaine natif complet "kringle 1" (K1).

2. Mutants de délétion de l'activateur de plasminogène tissulaire selon la revendication 1, lesdits mutants comprenant la chaîne native (L) et le domaine natif (K2), et étant dépourvus d'au moins une partie du domaine natif (F), d'au moins une partie du domaine du facteur de croissance épidermique natif (E) et du domaine natif complet (K1).

3. Mutants de délétion de l'activateur de plasminogène tissulaire selon la revendication 1, lesdits mutants comprenant la chaîne native (L), le domaine natif (F) et le domaine natif (K2), et étant dépourvus d'au moins une partie du domaine du facteur de croissance épidermique natif (E) et du domaine natif complet (K1).

4. Mutants de délétion de l'activateur de plasminogène tissulaire selon la revendication 1, lesdits mutants comprenant la chaîne native (L) et le domaine natif (F), et étant dépourvus d'au moins une partie du domaine du facteur de croissance épidermique natif (E), d'au moins une partie du domaine natif (K2) et du domaine natif complet (K1).

5. Information génétique de recombinaison sous forme d'ARN, d'ADN à brin unique ou d'ADN à double brin, codant soit un mutant de délétion de t-PA selon l'une quelconque des revendications 1 à 4, soit un précurseur d'un tel mutant de délétion de t-PA qui comprend le peptide signal natif (S), le propeptide natif (P), ou les deux, à la terminaison N.

6. Vecteur d'expression et/ou de clonage de recombinaison, comprenant un véhicule d'expression et/ou de clonage approprié, dans lequel est insérée l'information génétique de recombinaison selon la revendication 5.

7. Procédé pour préparer un mutant de délétion de t-PA selon l'une quelconque des revendications 1 à 4, en cultivant une culture cellulaire ou un micro-organisme hôte approprié, qui a été transformé par un vecteur d'expression de recombinaison comprenant un véhicule d'expression approprié, dans lequel est insérée l'information génétique de recombinaison codant ledit mutant de délétion de t-PA ou un précurseur de celui-ci qui comprend le peptide signal natif (S), le propeptide natif (P), ou les deux, à la terminaison N, et en isolant le mutant de délétion de t-PA qui y est produit.

8. Culture cellulaire ou micro-organisme qui a été transformé par un vecteur de recombinaison selon la revendication 6 et est capable de cloner et/ou d'exprimer l'information génétique de recombinaison selon la revendication 5.

9. Composition pharmaceutique utilisable dans la thérapie de la thrombose veineuse, de la thrombose artérielle, de l'infarctus du myocarde, de l'embolie pulmonaire, et d'autres conditions de circulation sanguine stoppée par des caillots de fibrine, comprenant un mutant de délétion de t-PA selon l'une quelconque des revendications 1 à 4, et au moins un véhicule, diluant ou support pharmaceutiquement acceptable.

10. Composition pharmaceutique selon la revendication 9, comprenant une solution aqueuse d'un mutant de délétion de t-PA selon l'une quelconque des revendications 1 à 4, qui est appropriée pour une administration intraveineuse, intra-artérielle ou intracoronarienne.

**Patentansprüche**

1. Gewebeplasminogen-Aktivator(t-PA)-Deletionsmutanten mit verbesserter Bindungsstärke, Bindungsselektivität oder beidem gegenüber Fibrin, wobei die Mutanten mindestens die native leichte Kette (L) und entweder die native Fingerdomäne (F) oder die native Kringel-2-Domäne (K2) oder beide enthalten, und denen mindestens die komplette native Kringel-1-Domäne (K1) fehlt.

2. Gewebeplasminogen-Aktivator-Deletionsmutant nach Anspruch 1, wobei die Mutanten die native L-Kette und die native K2-Domäne enthalten, und denen mindestens ein Teil der nativen F-Domäne, mindestens ein Teil der nativen epidermalen Wachstumsfaktordomäne (E) und die komplette native K1-Domäne fehlt.

3. Gewebeplasminogen-Aktivator-Deletionsmutant nach Anspruch 1, wobei die Mutanten die native L-Kette, die native F-Domäne und die native K2-Domäne enthalten, und denen mindestens ein Teil der nativen epidermalen Wachstumsfaktordomäne (E) und die komplette native K1-Domäne fehlt.

4. Gewebeplasminogen-Aktivator-Deletionsmutant nach Anspruch 1, wobei die Mutanten die native L-Kette und die native F-Domäne enthalten, und denen wenigstens ein Teil der nativen epidermalen Wachstumsfaktordomäne (E), mindestens ein Teil der nativen K2-Domäne und die komplette native K1-Domäne fehlt.

5. Rekombinante genetische Information in Form von RNA, einzelsträngiger DNA oder doppelsträngiger DNA, die entweder die t-PA Deletionsmutante nach einem der Ansprüche 1 bis 4 oder einen Vorläufer einer solchen t-PA Deletionsmutante kodiert, die das native Signalpeptid (S), das native Propeptid (P) oder beide am N-Terminus enthält.

6. Rekombinanter Klonierungs- und oder Expressionsvektor, enthaltend ein geeignetes Klonierungs- und/oder Expressionsvehikel mit einer darin eingeschlossenen rekombinanten genetischen Information nach Anspruch 5.

7. Verfahren zur Herstellung einer t-PA Deletionsmutante nach einem der Ansprüche 1 bis 4 durch Kultur eines geeignetes Wirtsmikroorganismus oder einer Zellkultur, die durch einen rekombinanten Expressionsvektor, der ein geeignetes Expressionsvehikel mit einer darin eingeschlossenen rekombinanten genetischen Information für die t-PA Deletionsmutante oder einen Vorläufer davon, die das native Signalpeptid (S), das native Propeptid (P) oder beides am N-Terminus enthält, transformiert wurde, und Isolierung der darin produzierten t-PA Deletionsmutante.

8. Mikroorganismus oder Zellkultur, der durch einen rekombinanten Vektor nach Anspruch 6 transformiert wurde, und zur Klonierung und/oder Expression der rekombinanten genetischen Information nach Anspruch 5 in der Lage ist.

9. Pharmazeutische Zusammensetzung zur Verwendung bei der Therapie von venöser Thrombose, arterieller Thrombose, myocardialem Infarkt, Lungenembolie und anderen Krankheitszuständen verursacht durch Blockierung des Blutkreislaufs durch Fibrinklumpen, enthaltend eine t-PA Deletionsmutante nach einem der Ansprüche 1 bis 4 und mindestens einen pharmakologisch annehmbaren Träger, Verdünner oder Vehikel.

10. Pharmazeutische Zusammensetzung nach Anspruch 9, enthaltend eine wäßrige Lösung einer t-PA Deletionsmutante nach einem der Ansprüche 1 bis 4, die geeignet ist zur intravenösen, intraarteriellen oder intracoronaren Verabreichung.

FIG.1

FIG. 2

time: hours →

absorbance 405nm →

EP 0 234 051 B1